# EUROPEAN PATENT APPLICATION

(11) **EP 2 898 882 A1**
(43) Date of publication of application: **29.07.2015**
(21) Application number: 13839349.1
(22) Date of filing: 17.09.2013
(51) Int. Cl.: A61K 31/325, A61K 9/70, A61P 25/28, A61P 25/00

(54) **MEDICINE HAVING IMPROVED RIVASTIGMINE STABILITY**

(30) Priority: 20.09.2012 KR 20120104727
(71) Applicant: SK Chemicals Co., Ltd., Seongnam-Si, Gyeonggi-Do 463-400 (KR)
(72) Inventor: Hwang, Yong-youn, Suwon-si Gyeonggi-do 440-210 (KR); Youn, Won-No, Seoul 151-711 (KR); Choi, Won-Jae, Seoul 138-878 (KR); Park, Yeo-Jin, Seoul 131-828 (KR); Oh, Joon-gyo, Suwon-si Gyeonggi-do 440-709 (KR); Kim, Sung-Hyuk, Suwon-si Gyeonggi-do 443-720 (KR); Kim, Hye-min, Anyang-si Gyeonggi-do 430-834 (KR); Shin, Sae byeok, Suwon-si Gyeonggi-do 441-440 (KR); Kim, Hun-Taek, Seoul 137-951 (KR); Sung, Jin Heung, Bucheon-si Gyeonggi-do 420-815 (KR)
(74) Representative: Graf von Stosch, Andreas
(86) International application number: PCT/KR2013/008426
(87) International publication number: WO 2014/046472

(57) **Abstract**

The present invention provides a rivastigmine medicine having improved stability. The invention also provides a method, for packing pharmaceutical medicines containing rivastigmine, capable of improving stability.

## Description

### TECHNICAL FIELD

The present disclosure relates to a drug product with improved stability of rivastigmine. Also, the present disclosure relates to a packaging method for improving stability of rivastigmine.

### BACKGROUND ART

Rivastigmine, which is (S)-N-ethyl-3-[1-dimethylamino)ethyl]-N-methyl-phenylcarbamate, is used for the treatment of Alzheimer's disease and is effective in inhibiting acetylcholinesterase in the central nervous system.

Rivastigmine is prepared in the formulations of patch. A transdermal composition in patch form is disclosed in example 2 of GB Patent No. 2,203,040. Such a patch is manufactured by mixing rivastigmine with two polymers and a plasticizer to prepare a viscous composition and applying the composition to a foil.

US Patent No. 6,335,031 relates to a transdermal composition containing rivastigmine or its salt, characterized by using a stabilizer. According to this invention, a transdermal composition containing rivastigmine is susceptible to degradation by an oxidation reaction with oxygen even though it is kept in air-tight condition, making it difficult to extend the shelf life for commercial transportation. To solve the problem, this invention discloses a transdermal composition of rivastigmine containing an antioxidant such as tocopherol and its ester, ascorbic acid, butylhydroxytoluene, butylhydroxyanisole, and propyl gallate. In commercial rivastigmine transdermal products, tocopherol is used as a stabilizer.

Like this, attempts have been continuously made to improve stability of a pharmaceutical preparation containing rivastigmine.

### DISCLOSURE

### Technical Problem

The present disclosure is directed to providing a drug product containing rivastigmine with improved stability.

The present disclosure is also directed to providing a packaging method of a pharmaceutical preparation containing rivastigmine to improve stability.

### Technical Solution

To achieve the objects, the present disclosure provides a drug product containing rivastigmine, in a packaged drug product comprising a pharmaceutical preparation containing rivastigmine, wherein an oxygen content in the packaging is less than 12 volumetric%, more preferably, less than or equal to 11 volumetric%, further more preferably, less than or equal to 10 volumetric%.

More preferably, the present disclosure provides, in the drug product according to the present disclosure, the drug product containing rivastigmine, wherein the pharmaceutical preparation is a rivastigmine free base containing patch.

The present disclosure also provides a method for improving stability of rivastigmine, in a method of packaging a pharmaceutical preparation containing rivastigmine, comprising adjusting an oxygen content to less than 12 volumetric%, more preferably, 11 volumetric% or less, further more preferably, 10 volumetric% or less, by substituting oxygen in a packaging with nitrogen.

More preferably, the present disclosure provides, in the method according to the present disclosure, the method for improving stability of rivastigmine, wherein the pharmaceutical preparation is a rivastigmine free base containing patch.

In the studies of various packaging methods for packaging a rivastigmine free base containing patch, the inventors made an invention difficult to anticipate that stability of rivastigmine is improved when reducing an oxygen concentration to less than 12%, preferably, 11% or less, most preferably, 10% or less, by substituting oxygen in a packaging with nitrogen.

### Advantageous Effects

The present disclosure provides a drug product containing rivastigmine with improved stability and a method for producing the drug product.

### BRIEF DESCRIPTION OF THE DRAWINGS

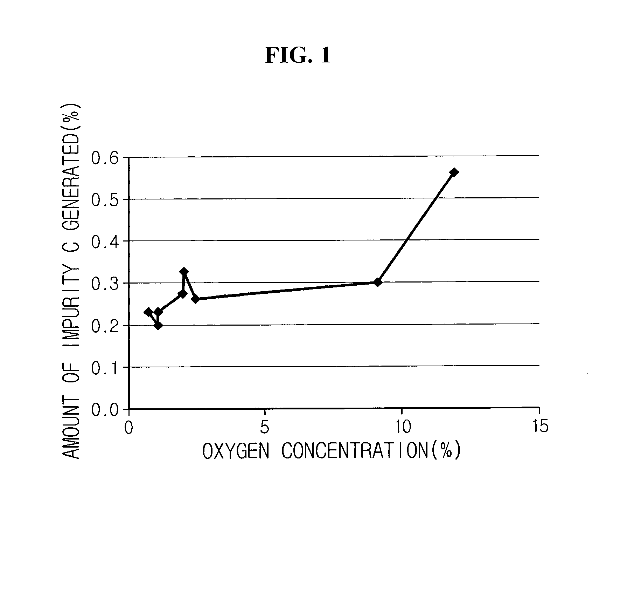

FIG. 1 is a graph showing an amount of degradation products or impurity C generated vs oxygen content in a packaging.

### MODE FOR CARRYING OUT THE INVENTION

Hereinafter, the present disclosure will be described in detail through embodiments to help the understanding of the present disclosure. However, the embodiments according to the present disclosure may be modified in a variety of different forms, and it should be understood that interpretation of the scope of the present disclosure is not limited to the following embodiments. The embodiments of the present disclosure are provided to person having ordinary skill in the art for the best explanation.

### <Manufacture of rivastigmine free base containing patch>

Based on the following table 1, a patch including a drug adhesive layer of rivastigmine free base and a skin adhesive layer was manufactured.

**[Table 1]**

| Drug adhesive layer | | Skin adhesive layer |
|---|---|---|
| Rivastigmine free base | 30 wt% | Silicone adhesive |
| Thickening agent | 20 wt% | |
| Acryl-based adhesive | 50 wt% | |

Specifically, a drug adhesive layer was formed by mixing rivastigmine free base, a thickening agent, and an acryl-based adhesive to prepare a drug adhesive layer solution, applying the drug adhesive layer solution to a silicone-coated polyester (PET) film, drying at 80°C for 10 minutes, and covering with a polyester (PET) film as a support layer. Aside from the drug adhesive layer, a skin adhesive layer was formed by applying/drying a silicone-based adhesive solution to a fluorine-coated polyester (PET) film in the same condition. The silicone-coated polyester (PET) film was removed from the drug adhesive layer and stacked on the skin adhesive layer, and then cut into circular pieces of 10 cm², to manufacture a rivastigmine free base containing patch.

### <Evaluation of oxygen concentration-dependent stability of rivastigmine free base patch in packaging>

The manufactured rivastigmine free base patch was packaged with a packaging made from a laminate of polyester and aluminum, and an oxygen concentration remaining in the packaging was measured by substituting air in the packaging with nitrogen. Subsequently, after 7-day storage of the packaged product in the condition of 60°C, an amount of degradation products of rivastigmine free base or impurity C generated versus the remaining oxygen concentration was measured. Impurity C is a degradation product resulting from oxidation, and its chemical name is 3-acetylpheny ethyl (methyl)carbamate.

An analysis condition of high performance liquid chromatography for measuring an amount of impurity C generated was as follows:
Injection volume: 10 µl
Column: RP18-C18, 250 mm X 4.6 mm, 5 µm
Detector: UV Detector (217 nm)
Flow rate: 1.0 mL / min
Run time: 30 minutes
Mobile phase: 10 mM Sodium-1-heptane sulphonate buffer: Acetonitrile (72:28)

Its result is shown in FIG. 1. As seen in FIG. 1, with the increasing oxygen concentration in air within a packaging, an amount of degradation products of rivastigmine or impurity C generated increased. There was no big change in the amount of degradation products of rivastigmine or impurity C generated until the oxygen concentration in air within the packaging reaches 10%, but when the oxygen concentration exceeds 10%, the amount of impurity C generated drastically increased.

According to the ICH guideline (Quality guidelines Q3B (R2)), requirements for limitation on degradation products found in complete drug products less than 0.5% are addressed.

## Claims

1. A drug product containing rivastigmine, in a packaged drug product comprising a pharmaceutical preparation containing rivastigmine, wherein an oxygen content in the packaging is less than 12%.

2. The drug product containing rivastigmine according to claim 1, wherein the oxygen content in the packaging is less than or equal to 11%.

3. The drug product containing rivastigmine according to claim 2, wherein the oxygen content in the packaging is less than or equal to 10%.

4. The drug product containing rivastigmine according to any one of claims 1 through 3, wherein the drug product comprises a rivastigmine free base containing patch as the pharmaceutical preparation.

5. A method for improving stability of rivastigmine, in a method of packaging a pharmaceutical preparation containing rivastigmine, comprising adjusting an oxygen content to less than 12% by substituting oxygen in a packaging with nitrogen.

6. The method for improving stability of rivastigmine according to claim 5, wherein the oxygen content in the packaging is adjusted to 11% or less.

7. The method for improving stability of rivastigmine according to claim 6, wherein the oxygen content in the packaging is adjusted to 10% or less.

8. The method for improving stability of rivastigmine according to any one of claims 5 through 7, wherein the method uses a rivastigmine free base containing patch as the pharmaceutical preparation.
